Europäisches Patentamt

(19) European Patent Office

Office européen des brevets

(11) Publication number: **0 158 602**

**A1**

## (12) EUROPEAN PATENT APPLICATION

(21) Application number: **85830021.3**

(22) Date of filing: **04.02.85**

(51) Int. Cl.⁴: **A 61 F 5/00**
**A 61 F 5/44**

(30) Priority: **10.02.84 IT 3556984 U**

(43) Date of publication of application:
**16.10.85 Bulletin 85/42**

(84) Designated Contracting States:
**AT BE CH DE FR GB LI LU NL SE**

(71) Applicant: **Consagra, Pietro**
**Via Cassia 1162**
**I-00189 Roma(IT)**

(72) Inventor: **Consagra, Pietro**
**Via Cassia 1162**
**I-00189 Roma(IT)**

(74) Representative: **Bazzichelli, Alfredo et al,**
**c/o Società Italiana Brevetti Piazza Poli 42**
**I-00187 Roma(IT)**

(54) A disposable device for allowing users thereof of female sex to urinate in a standing position.

(57) A device for allowing users thereof of female sex to urinate in a standing position comprises a central portion for receiving the urine and directing it from the rear to the front direction of the user and a pair of grasping lugs integral with said central portion along lateral crease lines, to be grasped by the hand, said device being made of flexible disposable web material.

FIG. 4

SPECIFICATION

BACKGROUND OF THE INVENTION

1.   Field of the invention

The present invention relates to a disposable sanitary device for allowing users thereof of female sex to urinate in a standing position.

The necessity for women to arrange themselves in a sitting or at least squatted position for the expulsion of urines entails, often, hygiene and practical  problems that render extremely troubling the accomplishment of said physiological operation. It is indeed well known that embarrassing situations arise when a woman has to use public W.C. for urinating, being sometimes these public W.C. extremely lacking in elementary hygiene.

SUMMARY OF THE INVENTION

Object of this invention is to provide a disposable device, such as to overcome the above mentioned inconveniences, shaped in such a way so as to allow women, particularly in emergency situations, to urinate in a standing position.

According to the invention, a disposable device of flexible impermeable web material for allowing users thereof of female sex to urinate in a standing position and to be held in operation between the thighs and under the pubic region of the users, comprises:

a central portion for receiving the urine and directing it from the rear to the front direction of the user, having a median crease line and a pair of lateral crease lines, said central portion having a rear projecting end of a rounded convex profile symmetrical to said median crease line, and a front projecting end of a profile converging toward said median crease line; and a pair of grasping lugs integral with said central portion along said lateral crease lines and to be grasped by the hand of the user.

In a first embodiment of the invention, said grasping lugs·have to be held each with one hand by the user and the device is so shaped as to fulfill this purpose.

As an alternative, the device is so designed as to be held with only one hand by the user in order to let the other hand free.

BRIEF DESCRIPTION OF THE DRAWINGS

The present invention will be better illustrated hereinafter with the disclosure of the embodiments thereof, given by way of non limitative example, and referring to the accompanying drawings, wherein:

figure 1 is a top plan view of a first embodiment of the device of the invention in a flat position;

figure 2 is an isometric view of the device of figure 1 in the operating position;

figure 3 is a top plan view of a second embodiment in a flat position; and

figure 4 is an isometric view of the embodiment of figure 3, in the operating position.

DESCRIPTION OF THE PREFERRED EMBODIMENTS

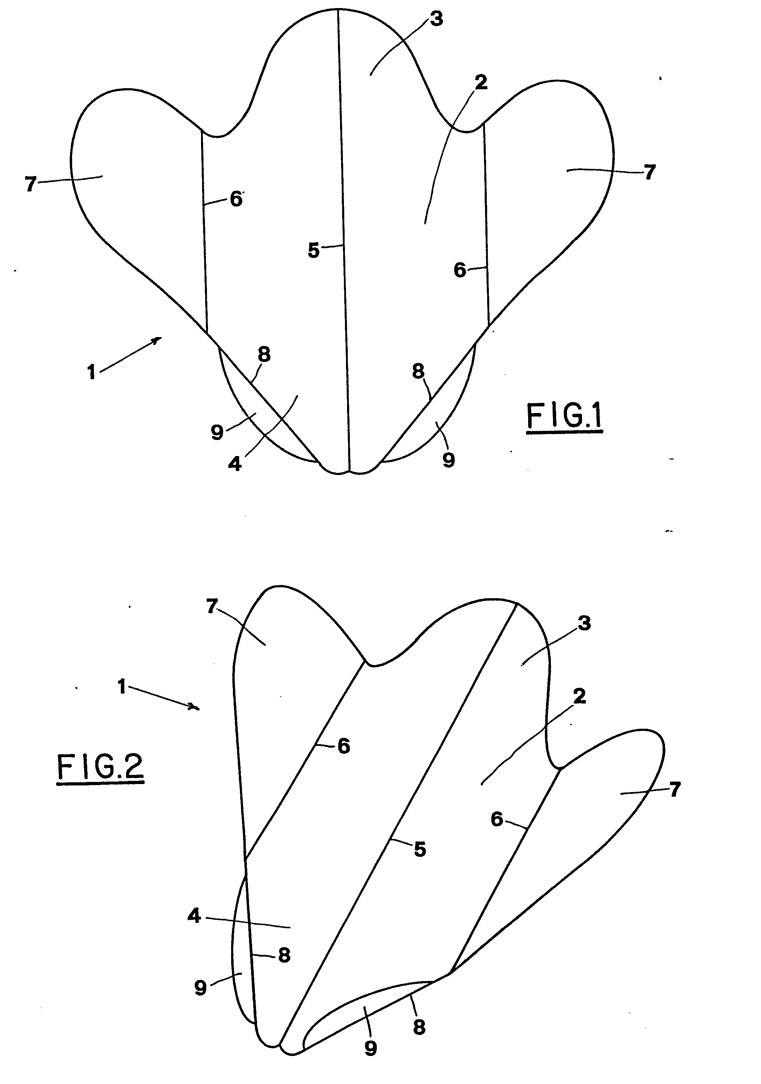

By making reference to figure 1, there is shown generally with reference numeral 1 the device according to the present invention comprising a relatively rigid impermeable sheet of biodegradable web material preferably a suitably treated paper sheet, comprising a central portion 2 with a rear projecting end 3 and a

- 3 -  0158602

front projecting end 4, opposed to the first one, for the discharge of the urine.

The front end 4 has a substantially triangular shape with two sides converging to the tip. The central portion 2 can be folded along a median crease line 5.

The lateral edges of the central portion 2 are united by means of lateral crease lines 6 to respective grasping lugs 7, while the sides of the front projecting end 4 are united by means of crease lines 8 to respective flaps 9 shaped as a circle segment.

As shown in figure 1 and 2, the rear end 3 is symmetrically rounded on both sides of the crease line 5 and the lugs 7 are so shaped as to merge into the profile of the central portion 2. Moreover, in the embodiment illustrated in figure 1 and 2, the median line 5 and the lateral lines 6 are substantially parallel.

As it is shown in figure 2, in order to set up the device according to the present invention in the operating position, one arranges for folding the central portion 2 along the median crease line 5, so that a substantially V-shaped channel is formed; the lugs 7 are folded upwards and inwards along the crease lines 6 in order to constitute respective parts for grasping with the fingers; then the flaps 9 are folded upwards along the crease lines 8 in order to create respective side walls on the front projecting end 4 for the discharge.

In use, the person using it grasps the device according to the present invention, in the operating position as above described and as shown in figure 2, by the lugs 7 with the thumb and the forefinger of the hands, and keeping it in downward inclined position, brings the rear projecting end 3 between the thighs under the pubic region so that the urine runs in the channel formed by the V-shaped folding of the central portion 2 along the median crease line 5, outcoming from the tip of the front projecting end 4 without overflowing laterally because of the flaps 9.

The device according to the present invention may be conveniently contained in a suitable small-dimensions envelope being it reduced to a minimum of overall dimensions by folding the lugs 7, along the crease lines 6, in contact with the respective parts of central portion 2, by folding the flaps 9, along the crease lines 8 in contact with the adjacent area of the front projecting end 4, and then the whole on itself along the median, crease line 5. In such a way, at least one device in question may be suitably contained, without any inconvenience for occupying space, within a lady purse in readiness for any evenience for which it is necessary.

As it can be seen from the above description, the device illustrated in figure 1 and 2 need to be held with the aid of the user's both hands.

Under certain circumstances it would be useful that one hand is left free for other purposes, particularly for holding garments, purses or the like.

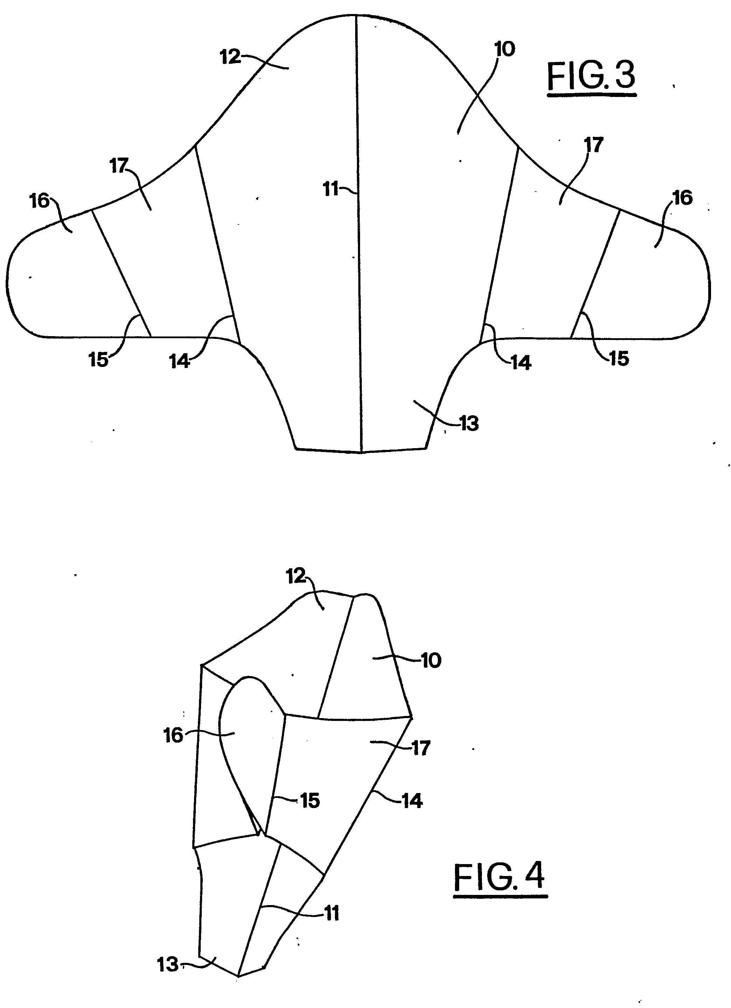

An embodiment of the invention which can be used with the aid of only one of the user's hands is illustrated in figure 3 and 4.

The embodiment of figure 3 and 4 shows the same main features as illustrated in figure 1 and 2, namely a central portion 10 provided with a median crease line 11, a rounded rear projecting end 12, a front projecting end 13 and lateral crease lines 14. The above mentioned elements have the same function as described with reference to figures 1 and 2.

In the present embodiment the grasping lugs united to the central portion 10 along the crease lines 14 are more elongated than the lugs 7 of figure 1 and 2 and they are provided with an additional crease line 15 spaced from the lateral crease line 14.

The crease line 15 defines an inner portion 17 and an outer portion 16 of the lug.

In operation, the inner portions 17 are folded up along the lateral crease lines 14 and the outer portions 16 are folded in the opposite direction, in order to bring together the outer portions 16, making it possible to hold the device by means of the use of one hand only, as shown in figure 4.

As a modification to the embodiment of figure 1 and 2, moreover the front projecting end 13 can be made free from the flaps 9 illustrated in figure 1 and 2, in which case the front end 13 terminates in a straight edge perpendicular to the median crease line 11, however maintaining the side edges converging toward the median line in the direction from rear to front.

As a further modification, the crease lines 14 and 15 can also be converging to the median line 11 rather than being parallel thereto.

Additional modifications can be envisaged within the scope of the present invention.

CLAIMS

1. A disposable device of flexible impermeable web material for allowing users thereof of female sex to urinate in a standing position and to be held in operation between the thighs and under the pubic region of the user, comprising:

a central portion for receiving the urine and directing it from the rear to the front direction of the user, having a median crease line and a pair of lateral crease lines, said central portion having a rear projecting end of a rounded convex profile symmetrical to said median crease line, and a front projecting end of a profile converging toward said median crease line; and

a pair of grasping lugs integral with said central portion along said lateral crease lines and to be grasped by the hand of the user.

2. A device according to claim 1, wherein said flexible impermeable web material is a biodegradable relatively stiff paper previously subjected to an impermeabilisation treatment.

3. A device according to claim 1, wherein said grasping lugs have an elongated, rounded shape merging into said rear convex profile and said front converging profile of said central portion.

4. A device according to claim 1, further comprising a pair of flaps integral with said central portion at said converging profile thereof along respective front crease lines, to be raised up in operation for directing the urine toward the tip of said front projecting end.

5. The device according to claim 4, wherein said lateral crease lines are parallel to said median crease line.

6. A device according to claim 1, wherein said grasping lugs have an additional crease line spaced from said lateral crease line, so as to form an inner portion and an outer portion of said lug, whereby folding said lugs at said lateral and additional crease lines results in bringing together said outer portions of the lugs in order to support said device in operating position by means of only one hand.

7. The device according to claim 6, wherein said lateral crease lines and said additional crease lines on the lugs are converging to said median crease line in the direction from rear to front.

8. The device according to claim 6, wherein said front projecting end has a front edge perpendicular to said median crease line.

1/2
0158602

FIG.1

FIG.2

0158602

FIG.3

FIG.4

**European Patent Office**

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | US-A-2 878 486  (D.E. BARTLETT et al .)  * Figures 1-5 * | 1,2,6,8 | A 61 F    5/00  A 61 F    5/44 |
| X | GB-A-1 132 820  (G.R. BJORK)  * Figures 1,2 * | 1-3 | |
| X | US-A-4 054 957  (H. DIAMOND)  * Abstract; figure 1 * | 1-3 | |
| X,P | GB-A-2 133 981  (S. BINI et al.)  * Abstract; figure 2 * | 1,2,4 | |
| Y | | 5 | |
| X | GB-A-2 002 629  (K. TOWFIGH)  * Abstract; figures 1-5 * | 1,2,6 | TECHNICAL FIELDS SEARCHED (Int. Cl.4) |
| Y | | 5 | A 61 F  A 61 B  A 61 G  B 67 C |
| X | US-A-3 815 581  (S. LEVIN)  * Figure 1 * | 1,2,6,8 | |
| A | | 3 | |
| | ---                -/- | | |

The present search report has been drawn up for all claims

| Place of search THE HAGUE | Date of completion of the search 19-04-1985 | Examiner WOLF C.H.S. |
|---|---|---|

## DOCUMENTS CONSIDERED TO BE RELEVANT

Page 2

| Category | Citation of document with indication, where appropriate, of relevant passages | Relevant to claim | CLASSIFICATION OF THE APPLICATION (Int. Cl.4) |
|---|---|---|---|
| X | US-A-4 023 216 (L.R. LI) <br> * Column 3, line 50 - column 4, line 41; figures 6-9 * | 1,2 | |
| A | | 3,4 | |
| A | --- <br> US-A-2 690 568 (M.C. WILLIS) | | |
| A | --- <br> US-A-3 579 653 (H.S. KUHN) | | |
| A | --- <br> US-A-3 731 869 (N.E. GRIFFIN) | | |
| A | --- <br> US-A-4 062 387 (H. PENICHE) | | TECHNICAL FIELDS SEARCHED (Int. Cl.4) |
| A | --- <br> US-A-3 804 134 (F.J. WEHKING) | | |
| A | --- <br> US-A-2 182 254 (C.B. FARRELL) | | |
| | ----- | | |

The present search report has been drawn up for all claims

| Place of search | Date of completion of the search | Examiner |
|---|---|---|
| THE HAGUE | 19-04-1985 | WOLF C.H.S. |